(19) Europäisches Patentamt
European Patent Office
Office européen des brevets

(11) EP 0 921 110 B1

(12) **FASCICULE DE BREVET EUROPEEN**

(45) Date de publication et mention de la délivrance du brevet:
**31.10.2001 Bulletin 2001/44**

(51) Int Cl.[7]: **C07C 45/72**, C07C 45/74, C07C 49/17, B01J 21/10, B01J 21/16

(21) Numéro de dépôt: **98402912.4**

(22) Date de dépôt: **23.11.1998**

(54) **Utilisation de composés de structure de type hydrotalcite pour la préparation de composés carbonyles-bèta-hydroxy et/ou alpha-bèta insaturés**

Verwendung von Verbindungen mit Hydrotalcitstruktur für die Herstellung von beta-Hydroxy und/oder alpha-beta ungesättigten Carbonylverbindungen

Use of compounds having hydrotalcite structure for the preparation of beta-hydroxy and/or alpha-beta unsaturated carbonyl compounds

(84) Etats contractants désignés:
**DE FR GB IT**

(30) Priorité: **05.12.1997 FR 9715390**

(43) Date de publication de la demande:
**09.06.1999 Bulletin 1999/23**

(73) Titulaire: **Atofina**
**92800 Puteaux (FR)**

(72) Inventeurs:
- **Teissier, Rémy**
  **69340 Francheville (FR)**
- **Tichit, Didier, Le Vallon des Sources**
  **34090 Montpellier (FR)**

(56) Documents cités:
**EP-A- 0 095 783**
**WO-A-92/00266**
**US-A- 5 254 743**

- **REICHLE W T: "Catalytic reactions by thermally activated, synthetic anionic clay minerals" J. CATAL. (JCTLA5,00219517);85; VOL.94 (2); PP.547-57, XP002072416 UNION CARBIDE CORP.;TECH. CENT.; BOUND BROOK; 08805; NJ; USA (US)**
- **KAGUNYA W ET AL: "Aldol condensation of acetaldehyde using calcined layered double hydroxides" APPL. CLAY SCI. (ACLSER,01691317);95; VOL.10 (1-2); PP.95-102, XP000610152 DEPARTMENT OF CHEMISTRY, UNIVERSITY OF CAMBRIDGE, LENSFIELD ROAD;CAMBRIDGE; CB2 1EW; UK (GB)**
- **SUZUKI E ET AL: "Aldol condensation reaction between formaldehyde and acetone over heat-treated synthetic hydrotalcite and hydrotalcite-like compounds" BULL. CHEM. SOC. JPN. (BCSJA8,00092673);88; VOL.61 (3); PP.1008-10, XP002072417 TOKYO INST. TECHNOL.;DEP. CHEM. ENG.; TOKYO; 152; JAPAN (JP)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 921 110 B1

Printed by Jouve, 75001 PARIS (FR)

## Description

La présente invention concerne l'utilisation de composés de structure de type hydrotalcite (HTLC) pour la préparation de composés carbonylés β-hydroxy etiou α-β insaturés par catalyse basique hétérogène.

Ces composés sont généralement obtenus industriellement par aldolisation et/ou aldolisation-crotonisation d'un aldéhyde et/ou d'une cétone en présence d'un catalyseur basique tel que les solutions diluées d'hydroxyde de sodium ou d'hydroxyde de potassium.

Cependant, l'utilisation de tels catalyseurs dans des procédés dits à catalyse homogène présentent de nombreux inconvénients. Notamment, avant de séparer les produits formés du milieu réactionnel il est nécessaire d'éliminer le catalyseur basique : par neutralisation avec un acide tel que $H_3PO_4$, précipitation des sels obtenus et filtration. Lors de ces traitements, on ne peut éviter l'encroûtement des colonnes à distiller par les sels résultant de la neutralisation, ce qui entraîne des arrêts réguliers de l'installation pour nettoyer les colonnes.

Afin de remédier à ces inconvénients, divers auteurs ont proposé d'opérer en catalyse hétérogène basique avec un solide facilement séparable de la phase organique ce qui permettait de simplifier le procédé et de supprimer les effluents provenant de la neutralisation de la soude.

C'est ainsi que Geng Zhang et al., Applied Catalysis, 36 (1988) 189-197 ont étudié l'aldolisation de l'acétone catalysée par des catalyseurs basiques solides tels que MgO, CaO, SrO, BaO, oxyde de lanthane III $La_2O_3$ et $ZrO_2$.

Ces auteurs ont trouvé que les activités de ces catalyseurs basées sur une même unité de surface spécifique étaient dans l'ordre : BaO > SrO> CaO> MgO> $La_2O_3$ > $ZrO_2$. De plus, pour MgO, l'addition d'eau et d'ammoniac par préabsorption entraînait une augmentation marquée de l'activité et de la sélectivité de l'obtention de DAA.

Kozo, Tanabe et al., Applied Catalysis, 48 (1989) 63-70, ont étudié l'addition des cations métalliques à l'oxyde de magnésium, pour obtenir un catalyseur d'aldolisation de l'acétone. L'influence des cations $Na^+$, $K^+$, $Rb^+$, $Cs^+$, $Al^{3+}$, $Mn^{2+}$, $Fe^{3+}$, $Co^{2+}$, $Ni^{2+}$, $Cu^{2+}$, $Zn^{2+}$ et $Zr^{4+}$ a été étudié. Ces auteurs ont remarqué que les cations $Na^+$, $Zr^{4+}$ et $Zn^{2+}$ augmentaient efficacement l'activité catalytique pour une teneur en poids de 0,5 à 1 % du cation métallique. L'addition d'eau en des quantités adéquates augmentait à la fois les activités et les sélectivités de ces catalyseurs MgO dopés à l'aide de ces cations. Par contre, ces auteurs ont mis en évidence que l'addition de n'importe quelle concentration de $Al^{3+}$ provoquait une diminution de l'activité, cette diminution allait dans le même sens que l'augmentation de la teneur en $Al^{3+}$. Le domaine étudié pour $Al^{3+}$, variant entre O % et environ 3 % d'$Al^{3+}$ en poids.

Le brevet US 5,144,089 divulgue un procédé de condensation aldolique en phase liquide, notamment la conversion du butanal en 2-éthyl 2-hexènal en présence d'un catalyseur solide. Le catalyseur est une solution solide d'oxyde de magnésium et d'oxyde d'aluminium dérivée d'une hydrotalcite et ayant une surface spécifique supérieure à $250 m^2/g$.

Le brevet français FR 2729137 décrit un procédé d'aldolisation sélective de l'acétone en diacétonealcool en présence d'un catalyseur basique solide de formule :

$$[(Mg^{2+})_{1-x} (Al^{3+})_x (OH^-)_{2+x}], mH_2O$$

avec $0,20 \le x \le 0,33$ et $m < 1$.

Ces catalyseurs permettent une bonne conversion en diacétonealcool, qui égale pratiquement la valeur à l'équilibre thermodynamique à 0°C.

On a maintenant trouvé que l'on pouvait obtenir des composés carbonylés β-hydroxy et/ou α-β insaturés avec des conversions élevées en utilisant comme catalyseur basique un composé de formule :

$$[M(II)_{1-x}, M(III)_x (OH)_{2+x}], m(H_2O) \quad (I)$$

dans laquelle M(II) représente un cation divalent des métaux choisis dans le groupe constitué par le nickel, le zinc, le cobalt le magnésium ou le mélange d'au moins deux des métaux précités, M(III) représente un cation trivalent des métaux choisis dans le groupe constitué par l'aluminium, le fer, le gallium, le chrome, à l'exclusion du composé de formule (I) dans laquelle M(II) est le magnésium et M(lll) est l'aluminium, $0,20 \le x \le 0,33$ et $m \le 1$.

Selon la présente invention, on préfère utiliser des composés de formule (I) dans lesquels M(ll) est le nickel ou le zinc et M(lll) est l'aluminium ou le gallium.

Selon la présente invention, on préfère également utiliser un composé de formule (I) dans lequel M(II) est le magnésium et M(III) est le gallium.

Ces composés peuvent être préparés selon un protocole équivalent à celui décrit dans le brevet FR 2729137 incorporé dans la présente par référence.

Dans une première étape, on prépare un composé minéral de type hydrotalcite synthétique de formule :

$$M(II)_{1-x}\ M(III)_x\ (OH)_2\ (CO_3)_{x/2},\ mH_2O$$

selon des méthodes connues dérivées des méthodes mises au point par S.Myata (Clay and Clays Minerais, 1980, 28, pages 50-56) ou Reichle (EP 95783).

Il s'agit de faire coprécipiter un sel du métal divalent avec un sel du métal trivalent dans une solution au pH contrôlé et alcalin : 8 < pH < 11. Le pH est ajusté grâce à une solution de carbonate de sodium et/ou de soude.

Pour finir la précipitation, on peut chauffer le gel au reflux durant plusieurs heures, le solide est séparé par filtration, lavé et séché.

Le solide ainsi obtenu contient comme anion intercalaire surtout du $CO_3{}^{2-}$ et d'autres anions minéraux des sels utilisés pour la synthèse tels que $Cl^-$, $NO^-_3$, $SO_4{}^{2-}$. Ces derniers sont éliminés par échange avec du $CO_3{}^{2-}$ en milieu aqueux.

Le produit ainsi échangé ne doit plus contenir comme anion intercalaire que le carbonate (vérifié par analyse chimique).

Ce solide est calciné sous courant gazeux sec, généralement l'air, pendant une durée comprise entre 2 h et 36 h, de préférence 8 h et 15 h, à une température au plus égale à 800°C et, de préférence comprise entre 400°C et 600°C. La température de calcination dépend de la nature chimique du solide, c'est-à-dire la nature des métaux. Elle est choisie pour chaque solide sur deux critères :

- obtention de l'oxyde mixte amorphe exempt de l'anion carbonate,
- possibilité de reconstruction de la structure.

En effet, si on calcine trop haut, il y a séparation entre les phases de l'oxyde du métal divalent et de l'oxyde du métal trivalent. Cette séparation peut être invisible en diffraction X et ne se voir qu'au cours de la reconstruction de la structure lamellaire.

Ensuite, on fait subir au produit calciné une étape de ré hydratation qui a pour but de reconstruire la structure lamellaire.

Celle-ci se fait en présence d'eau. Cette quantité est toujours inférieure à la quantité théorique pour neutraliser les charges positives des feuillets. Chaque atome de M(III) apporte une charge positive. Plus précisément, la réaction de réhydratation peut s'écrire de la facon suivante :

$$(1-x)M(II)O,\ x/2\ M(III)_2O_3 + (1 + x/2 + m)H_2O \rightarrow$$

$$[M(II)_{1-x}\ M(III)_x\ (OH)_{2+x}],\ mH_2O$$

La réhydratation se fait à une température comprise entre O°C et 200°C. Au delà de 100°C, la réhydratation est conduite en autoclave sous pression autogène ou sous pression initiale d'azote, à température ambiante, comprise entre 0.1 MPa (1 bar) et 1 MPa (10 bar).

Elle se fait sur une durée comprise entre une demi-heure et 120 h selon la difficulté de reconstruction de la structure lamellaire.

L'eau nécessaire à la reconstruction peut être sous forme gazeuse. La réhydratation peut être conduite en lit fixe traversé ou en lit fluidisé. Les débits sont évidemment différents. Le temps de réhydratation est aussi différent. Le débit gazeux est variable selon la quantité de solide à activer.

L'eau de réhydratation peut être sous forme liquide. Il s'agit alors de traiter le solide, oxyde mixte amorphe par de l'eau. Soit, on réalise une suspension de solide dans l'eau, soit on laisse le solide sous eau.

La cinétique de réhydration dépend de la température du milieu, de l'agitation, de la granulométrie du solide, et de la nature des métaux constituant le solide. Ainsi, la réhydratation peut être achevée en une heure ou plusieurs jours.

En effet, un oxyde mixte magnésium-gallium s'hydrate en une heure à 60°C ; son homologue zinc-aluminium s'hydrate à 80°C en 8 heures ; un oxyde mixte nickel aluminium pour se réhydrater partiellement, a besoin d'un traitement de 48 heures à 120°C.

Si on réalise le spectre de diffraction X des différents solides, on constate :

- avant calcination le spectre d'une hydrotalcite,
- après calcination, la disparition des raies caractéristiques de la structure hydrotalcite, l'apparition de la raie large

et peu intense de l'oxyde du métal divalent amorphe.
- après réhydratation, la réapparition des raies de la structure hydrotalcite et la diminution, voire dans les meilleurs cas, la disparition de la raie de l'oxyde du métal divalent.

Les composés de formule (I) sont utilisés pour la préparation de composés carbonylés β-hydroxy et/ou α-β insaturés par catalyse basique.

A cet effet, on peut faire réagir sur lui-même, en présence d'un composé de formule (I), un aldehyde de formule générale $R^1$-CHO, dans laquelle $R^1$ représente un radical alkyle linéaire ou ramifié contenant de 1 à 10 atomes de carbone ou avec au moins un composé choisi parmi un autre aldéhyde de formule générale $R^2$-CHO, dans laquelle $R^2$ représente un atome d'hydrogène, un radical alkyle linéaire ou ramifié ou cyclique contenant de 1 à 10 atomes de carbone, un radical phényle, un radical benzyle, un radical aralkyle etiou avec une cétone de formule générale $R^5$-CO-$R^6$, dans laquelle $R^5$ et $R^6$ identiques ou différents représentent chacun un radical alkyle linéaire ou ramifié contenant de 1 à 10 atomes de carbone et pouvant être reliés entre eux pour former un cycle.

On peut également faire réagir sur elle-même, en présence d'un composé de formule (I) une cétone de formule générale $R^3$-CO-$R^4$ dans laquelle R3 et $R^4$ identiques ou différents représentent chacun un radical alkyle linéaire ou ramifié contenant de 1 à 10 atomes de carbone, ou bien avec au moins un composé choisi parmi un aldéhyde de formule générale $R^2$-CHO, dans laquelle $R^2$ a la même signification que précédemment, et/ou avec une autre cétone de formule $R^5$-CO-$R^5$, dans laquelle $R^5$ et $R^6$ identiques ou différents ont la même signification que précédemment.

La cétone $R^3$-CO-$R^4$ est de préférence choisie parmi celles dans lesquelles $R^3$ ou $R^4$ représente un radical méthyle comme notamment l'acétone, l'éthylméthylcétone ou la méthylpropylcétone.

L'aldéhyde $R^1$ - CHO est avantageusement choisi parmi l'éthanal, le propanal, le butanal, l'isobutanal, le pentanal, l'hexènal et l'heptènal.

L'aldéhyde $R^2$ - CHO est avantageusement choisi parmi le méthanal, l'éthanal, le propanal, le butanal, l'isobutanal et le benzaldéhyde. De préférence, on utilise le méthanal, le butanal et l'isobutanal.

Les composés de formule (I) sont utilisés notamment pour la préparation de composés carbonylés β-hydroxy et α-β insaturés tels que l'oxyde de mesityle, le 2-éthyl 2-hexénal, le 5-méthyl 3 hexèn-2-one, le 3-heptèn-2-one, le 2,2'4-trimethyl 3-ol pentanal, le 2-éthyl-3- ol héxanal et tout particulièrement pour la préparation du diacétonealcool (4-hydroxy-4 méthyl-2 pentanone) à partir de l'acétone par catalyse basique hétérogène selon la réaction :

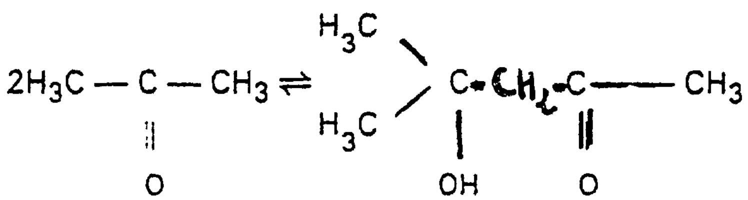

C'est une réaction équilibrée, la concentration à l'équilibre à 0°C est de 23,1 % et diminue lorsque la température augmente.

En outre, lorsque la température augmente la sélectivité en diacétonealcool baisse suite à des réactions de déshydratation et de polycondensation, lesdites réactions conduisant à la formation d'oxyde de mésityle, de tridiacétonealcool et de phorone.

Les composés carbonylés β-hydroxy et/ou α-β insaturés peuvent être obtenus en continu ou en discontinu selon des conditions opératoires (températures, pressions) connues de l'homme du métier.

Lorsqu'on opère en discontinu, la quantité de composé (I) selon l'invention utilisée par rapport à la charge totale de réactifs introduits dans le réacteur est en général comprise entre environ 0,5 % et environ 20 %, et de préférence comprise entre environ 2 % et environ 15 %.

Un rapport massique de composé (I) utilisé sur la charge totale de réactifs introduits, compris entre environ 3 % et environ 10 % est particulièrement préféré.

S'agissant de l'obtention du diacétonealcool, on peut opère en lit fixe ou en lit agité à une température réduite de façon à favoriser la formation du diacétonealcool. On opérera à une température au plus égale à 10°C et, de préférence comprise entre 0°C et 5°C.

Dans l'éventualité où l'on opère en lit agité, on utilisera des quantités pondérales de composé de formule (I) allant de 5 % à 10 % par rapport à l'acétone mise en oeuvre.

L'utilisation des composés de formule (I) en tant que catalyseurs basiques présentent l'avantage de conduire sélectivement à des composés carbonylés β-hydroxy et/ou α-β insaturés avec une conversion élevée.

Les exemples qui suivent illustrent l'invention.

**PREPARATION D'UNE HYDROTALCITE DE NICKEL ET D'ALUMINIUM ACTIVEE.**

**Synthèse de l'hydrotalcite :**

Dans un réacteur de deux litres, ouvert à l'air, muni d'une agitation et d'un condenseur à reflux, on introduit sous agitation et à température ambiante 600 ml d'une solution aqueuse de bicarbonate de sodium. La quantité de bicarbonate est contrôlée pour obtenir un pH de 8. Puis, on introduit à l'aide d'une pompe, une solution aqueuse de 400 ml contenant 0,3 mole de nitrate de nickel et 0,1 mole de chlorure d'aluminium. Le débit de la pompe est compris entre 3 et 6 ml/min. Durant cette addition le pH est maintenu à 8 par ajout d'une solution de soude 3N.
En fin d'introduction, on porte le mélange au reflux (100°C) toujours sous agitation. On maintient le reflux durant 18 heures environ.
Puis on refroidit, on filtre le solide, on le lave à l'eau jusqu'à ce que les eaux de lavages soient exemptes de chlorures.
Le produit obtenu est vert et a la formule suivante :

$$Ni_{0,75}Al_{0,25}(OH)_2(CO_3{}^{2-})_{0,172}, mH_2O$$

**Echange au carbonate :**

Ce produit est échangé comme suit : à une solution de 0,69 g de $Na_2CO_3$ dans 100 ml d'eau distillée, on ajoute deux grammes d'hydrotalcite préparée plus haut. Sous agitation, on porte !a suspension à 80°C durant trois heures. On filtre, puis on lave deux fois à l'eau. On effectue un second échange de la même manière. On obtient un solide où il est impossible de titrer le chlorure par potentiométrie (argentimétrie en milieu nitrique où l'hydrotalcite est soluble).

**Calcination :**

On calcine le solide échangé sous air sec selon le programme thermique suivant :

- montée à 450°C en 5 heures,
- palier à 450°C pendant 10 heures,
- refroidissement libre toujours sous balayage à l'air sec.

Le poids de solide calciné représente 60 à 65 % du poids de solide initial.
L'oxyde mixte est noir.

**Activation** :

Le solide est mis en suspension dans 100 g d'eau décarbonatée puis la suspension est introduite dans un autoclave chemisé en Téflon. La suspension est portée à 120°C durant 48 heures.
Le solide est récupéré vert.
On obtient un composé de formule :

$$Ni_{0,75}Al_{0,25}(OH)_{2,25}, mH_2O$$

**PREPARATION D'UNE HYDROTALCITE DE ZINC ET D'ALUMINIUM ACTIVEE.**

**Synthèse de l'hydrotalcite** :

Dans un réacteur ouvert de deux litres, muni d'une agitation et d'un condenseur à reflux, on introduit sous agitation et à température ambiante 600 ml d'une solution aqueuse de bicarbonate de sodium. La quantité de bicarbonate est contrôlée pour obtenir un pH de 8. Puis, on introduit à l'aide d'une pompe, une solution aqueuse de 400 ml contenant 0,3 mole de chlorure de zinc et 0,1 mole de chlorure d'aluminium. Le débit de la pompe est compris entre 3 et 6 ml/mn. Durant cette addition le pH est régulé à 8 par ajout d'une solution de soude 3N.

En fin d'introduction, on porte le mélange au reflux (100°C) toujours sous agitation. On maintient le reflux durant 18 heures environ.

Puis on refroidit, on filtre le solide, on le lave à l'eau jusqu'à ce que les eaux de lavages soient exemptes de chlorures.

Le produit obtenu a la formule suivante :

$$Zn_{0,76}Al_{0,24}(OH)_2(CO_3^{2-})_{0,14}, mH_2O$$

**Echange au carbonate :**

Ce produit est échangé deux fois comme suit : à une solution de 0,69 g de $Na_2CO_3$ dans 100 ml d'eau distillée, on ajoute deux grammes d'hydrotalcite préparée ci-avant. Sous agitation, on porte la suspension à 80°C durant trois heures. On filtre, puis on lave deux fois à l'eau. On effectue un second échange de la même manière. On obtient un solide où il est impossible de titrer le chlorure par potentiométrie.

**Calcination :**

On calcine le solide échangé sous air sec selon le programme thermique suivant :

- montée à 450°C en 5 heures,
- palier à 450°C pendant 10 heures,
- refroidissement libre toujours sous balayage à l'air sec.

**Activation :**

Le solide est mis en suspension dans 100 g d'eau décarbonatée à 80°C. La suspension est maintenue à 80°C durant 8 heures.

On obtient un composé de formule :

$$Zn_{0,76}Al_{0,24}(OH)_{2,24}, mH_2O$$

**PREPARATION D'UNE HYDROTALCITE DE NICKEL ET DE GALLIUM ACTIVEE**

**Synthèse de l'hydrotalcite :**

Elle est réalisée selon des conditions identiques à celles utilisées pour préparer les hydrotalcites précédentes.

Le produit obtenu a la formule suivante :

$$Ni_{0,78} Ga_{0,22}(OH)_2 (CO_3^{2-})_{0,146} 0,76H_2O$$

L'échange au carbonate, la calcination et l'activation sont réalisés selon les mêmes conditions identiques à celles utilisées pour préparer les hydrotalcites de zinc ou de nickel et d'aluminium activées.

On obtient un composé de formule :

$$Ni_{0,78}Ga_{0,22}(OH)_{2,22}, mH_2O$$

**UTILISATION DES HYDROTALCITES ACTIVEES PRECEDEMMENT PREPAREES POUR LA SYNTHESE DU DIACETONEALCOOL :**

*ESSAI 1*

Dans un réacteur de 500 ml agité, muni d'un condenseur à reflux, d'un système d'inertage à l'azote et d'une double enveloppe permettant de le thermostaté par circulation d'un fluide thermique, on introduit 100 g d'acétone. Sous agitation, on porte l'acétone à 0°C en purgeant le ciel du réacteur à l'azote.

Lorsque la température est stabilisée, on introduit 3 g d'hydrotalcite de nickel et d'aluminium activée précédemment préparée humide en évitant au maximum tout contact de celle-ci avec l'air.
On rince par 100 g d'acétone quatre fois.
On laisse réagir sous agitation. On prélève régulièrement un échantillon que l'on analyse en chromatographie phase gazeuse (CPG) sur un Chromatographe Hewlett-Packard 6890. On suit ainsi la formation de diacétonealcool, d'oxyde de mésityle et de triadiacétonealcool.
Les caractéristiques de l'analyse CPG sont les suivantes :

- colonne capillaire ayant une longueur de 30 m et un diamètre de 1,33 µm, Hewlett-Packard Carbowax 20 M ;
- détecteur ; catharomètre,
- gaz vecteur : hélium (débit : 20 ml/s) ;
- injecteur : température 150°C,
- détecteur ; température 180°C,
- température de la colonne : 5 minutes à 50°C puis 8°C/min jusqu'à 200°C.

Selon ces conditions analytiques, on ne détecte ni oxyde de mésityle, ni tridiacétonedialcool.
On reporte dans le tableau 1 ci-après le pourcentage pondéral de diacétone alcool (DAA) obtenu en fonction de la durée de réaction.

TABLEAU 1

| DUREE (MIN) | % DE DAA |
|---|---|
| 0 | 0 |
| 20 | 6,5 |
| 60 | 10,5 |
| 120 | 12,9 |
| 180 | 14,5 |
| 240 | 15,45 |
| 1320 | 20,4 |

*ESSAI 2*

On opère comme dans l'essai 1 excepté que l'on utilise 2,8 g d'hydrotalcite de zinc et d'aluminium activée tel que précédemment préparée.
Dans le tableau 2, on reporte le pourcentage pondéral de diacétone alcool (DAA) obtenu en fonction de la durée de réaction.

TABLEAU 2

| DUREE (MIN) | % DE DAA |
|---|---|
| 0 | 0 |
| 20 | 0,33 |
| 240 | 1,8 |
| 1320 | 4,3 |

Il y a lieu de noter que dans cet essai également , l'oxyde de mesityle et le triadiacétonealcool n'ont pas été détectés dans les conditions d'analyse CPG telles que mentionnées précédemment.

*ESSAI 3*

On opère comme dans l'essai 1 excepté que l'on utilise 2,8 g d'hydrotalcite de nickel et de gallium activée tel que précédemment préparée.
Dans le tableau 3, on reporte le pourcentage pondéral de diacétone alcool (DAA) obtenu en fonction de la durée de réaction.

TABLEAU 3

| DUREE (MIN) | % DE DAA |
|---|---|
| 0 | 0 |
| 20 | 8,5 |
| 60 | 12,6 |
| 120 | 14,3 |
| 180 | 16,3 |
| 1320 | 21 ,1 |

**Essai de recyclage :**

L'hydrotalcite de nickel et d'aluminium utilisée dans l'essai 1 a été réutilisée une fois pour la synthèse du diacétone alcool selon des conditions identiques à celles de l'essai 1, avec une masse d'hydrotalcite de nickel et d'aluminium de 3 g, comme dans l'essai 1.

Les résultats sont reportés dans le tableau 4. Dans ce tableau, on reporte le pourcentage pondéral du diacétone alcool (DAA) obtenu en fonction de la durée de réaction avec l'hydrotalcite de nickel et d'aluminium utilisé dans l'essai 1 (essai 1) et avec la même hydrotalcite réutilisée une fois (recyclage).

TABLEAU 4

| DUREE (MIN) | % DE DAA | |
|---|---|---|
| | ESSAI 1 | RECYCLAGE |
| 0 | 0 | 1,5 (1) |
| 20 | 6,5 | 7,5 |
| 60 | 10,5 | 11 |
| 120 | 12,9 | 13 |
| 180 | 14,5 | non mesuré |
| 240 | 15,45 | 15,6 |
| 1320 | 20,4 | |

(1) La valeur initiale n'est pas nulle, car l'essai de recyclage est réalisé avec le pied de l'esai 1 (catalyseur plus milieu réactionnel plus 100 g d'acétone fraîche).

**On constate que le recyclage n'entraîne pas de perte d'activité du catalyseur.**

## Revendications

1. Utilisation pour la préparation de composés carbonylés β-hydroxy et/ou α-β insaturés d'un composé de structure de type hydrotalcite de formule :

$$[M(II)_{1-x}, M(III)_x (OH)_{2+x}], m(H_2O) \quad (I)$$

dans laquelle M(II) représente un cation divalent choisi dans le groupe constitué par le nickel, le zinc, le cobalt, le magnésium ou le mélange de deux ou plus des métaux précitées M(III) représente un cation trivalent choisi dans le groupe constitué par l'aluminium, le gallium, le fer, le chrome, à l'exclusion du composé de formule (I) dans laquelle M(II) est le magnésium et M (III) est l'aluminium, x est compris entre 0,20 et 0,33 et m ≤ 1.

2. Utilisation selon la revendication 1, **caractérisée en ce que** dans le composé de formule (I), M(II) est le nickel ou le zinc et M(III) est l'aluminium ou le gallium.

3. Utilisation selon l'une des revendications 1 ou 2, **caractérisée en ce que** dans le composé de formule (I) M(ll) est le nickel et M(III) est l'aluminium.

4. Utilisation selon l'une des revendications 1 ou 2, **caractérisée en ce que** dans le composé de formule (I) M(ll) est le zinc et M(III) est l'aluminium.

5. Utilisation selon l'une des revendications 1 ou 2, **caractérisée en ce que** dans le composé de formule (I) M(ll) est le nickel et M(III) est le gallium.

6. Utilisation selon la revendication 1, **caractérisée en ce que** dans le composé de formule (I), M(ll) est le magnésium et M(III) est le gallium.

7. Utilisation d'un composé de formule (I) selon l'une quelconque des revendications 1 à 6 pour l'aldolisation de l'acétone en diacétonealcool.

### Patentansprüche

1. Verwendung einer Struktur vom Hydrotalcittyp der Formel:

$$[M(II)_{1-x}, M(III)_x (OH)_{2+x}], m(H_2O) \quad (I)$$

in der M(II) ein zweiwertiges Kation, gewählt aus der Gruppe von Nickel, Zink, Kobalt, Magnesium oder einem Gemisch von zwei oder mehreren der genannten Metalle; M(III) ein dreiwertiges Kation ausgewählt aus der Gruppe von Aluminium, Gallium, Eisen, Chrom ist, ausgenommen die Verbindung der Formel (I), in der M(II) Magnesium und M(III) Aluminium ist, x zwischen 0,20 und 0,33 liegt und $m \leq 1$ ist, zur Herstellung von β-Hydroxylcarbonyl und/oder α-β ungesättigten Verbindungen.

2. Verwendung nach Anspruch 1, **dadurch gekennzeichnet, daß** in der Verbindung der Formel (I) M(II) Nickel oder Zink und M(III) Aluminium oder Gallium ist.

3. Verwendung nach einem der Ansprüche 1 oder 2, **dadurch gekennzeichnet, daß** in der Verbindung der Formel (I) M(II) Nickel und M(III) Aluminium ist.

4. Verwendung nach einem der Ansprüche 1 oder 2, **dadurch gekennzeichnet, daß** in der Verbindung der Formel (I) M(II) Zink und M(III) Aluminium ist.

5. Verwendung nach einem der Ansprüche 1 oder 2, **dadurch gekennzeichnet, daß** in der Verbindung der Formel (I) M(II) Nickel und M(III) Gallium ist.

6. Verwendung nach Anspruch 1, **dadurch gekennzeichnet, daß** in der Verbindung der Formel (I) M(II) Magnesium und M(III) Gallium ist.

7. Verwendung einer Verbindung der Formel (I) nach einem der Ansprüche 1 bis 6 für die Aldolisierung von Aceton zu Diacetonalkohol.

### Claims

1. Use in the preparation of β-hydroxycarbonyl and/or α,β-unsaturated carbonyl compounds of a compound with a structure of hydrotalcite type of formula:

$$[M(II)_{1-x}M(III)_x(OH)_{2+x}]\cdot mH_2O \quad (I)$$

in which M(II) represents a divalent cation chosen from the group consisting of nickel, zinc, cobalt, magnesium or

the mixture of two or more of the abovementioned metals; M(III) represents a trivalent cation chosen from the group consisting of aluminium, gallium, iron or chromium, with the exception of the compound of formula (I) in which M(II) is magnesium and M(III) is aluminium, x is between 0.20 and 0.33 and $m \leq 1$.

**2.** Use according to Claim 1, **characterized in that**, in the compound of formula (I), M(II) is nickel or zinc and M(III) is aluminium or gallium.

**3.** Use according to either of Claims 1 and 2,
**characterized in that**, in the compound of formula (I), M(II) is nickel and M(III) is aluminium.

**4.** Use according to either of Claims 1 and 2,
**characterized in that**, in the compound of formula (I), M(II) is zinc and M(III) is aluminium.

**5.** Use according to either of Claims 1 and 2,
**characterized in that**, in the compound of formula (I), M(II) is nickel and M(III) is gallium.

**6.** Use according to Claim 1, **characterized in that**, in the compound of formula (I), M(II) is magnesium and M(III) is gallium.

**7.** Use of a compound of formula (I) according to any one of Claims 1 to 6 in the aldolization of acetone to diacetone alcohol.